# EUROPÄISCHE PATENTSCHRIFT

(11) **EP 0 673 434 B1**
(45) Veröffentlichungstag und Bekanntmachung des Hinweises auf die Patenterteilung: **28.07.1999**
(21) Anmeldenummer: 93924519.7
(22) Anmeldetag: 12.11.1993
(51) Int. Cl.: C12Q 1/58, B01L 3/00, A61B 10/00

(54) **VORRICHTUNG ZUR DURCHFÜHRUNG KOMBINIERTER UREASETESTS VON ANTRUM/KORPUS-BIOPSIEN ZUR DIAGNOSE VON GASTROINTESTINALERKRANKUNGEN**
DEVICE FOR CARRYING OUT UREASE TESTS FOR COMBINED ANTRUM/CORPUS BIOPSIES TO DIAGNOSE GASTRO-INTESTINAL ILLNESSES
DISPOSITIF POUR L'EXECUTION DE TESTS AVEC L'UREASE SUR DES BIOPSIES COMBINEES DE L'ANTRE ET DU CORPS POUR LE DIAGNOSTIC DES MALADIES GASTRO-INTESTINALES

(30) Priorität: 08.12.1992 DE 9217130 U
(43) Veröffentlichungstag der Anmeldung: 27.09.1995
(73) Patentinhaber: Astra Aktiebolag, 151 85 Södertälje (SE)
(72) Erfinder: HECKENMÜLLER, Harald, D-22559 Hamburg (DE); MEYER, Hansjörg, D-25436 Uetersen (DE)
(74) Vertreter: Weber, Dieter, Dr.
(86) Internationale Anmeldenummer: DE9301085
(87) Internationale Veröffentlichungsnummer: WO9413830

(56) Entgegenhaltungen:
- EP-A- 0 204 438
- EP-A- 0 369 292
- WO-A-91/17832
- DE-A- 3 531 481
- FR-A- 2 618 553
- US-A- 4 721 679
- ACTA THERAPEUTICA Bd. 14, Nr. 3 , 1988 , BRÜSSEL, BE Seiten 205 - 214 P. MALFERTHEINER ET AL. 'Campylobacter-Urease-Test (CUT) in der Diagnostik der chronischen Gastritis' in der Anmeldung erwähnt
- DATABASE WPI Section Ch, Week 8631, Derwent Publications Ltd., London, GB; Class J04, AN 86-202681 & JP,A,57 113 364 (TSUNEMITSU KK) 14. Juli 1982
- IMMUNITÄT UND INFEKTION Bd. 17, Nr. 3, Juni 1989, BADEN-BADEN, DE, Seiten 83 - 90 G. BÖRSCH ET AL. 'Campylobacter pylori: Klinische Korrelationen und prospektive vergleichende Untersuchungen zur Wertigkeit mehrerer Testverfahren'

## Beschreibung

Die Erfindung betrifft eine Vorrichtung zur Durchführung von Ureasetests kombinierter Antrum/Korpus-Biopsien zur Diagnose von Gastrointestinalerkrankungen.

Gastrointestinalerkrankungen, wie z.B. die duodenale und auch gastrale Ulkuskrankheit, vor allem aber die chronisch aktive Gastritis, sind eng mit der teilweisen oder auch diffusen Besiedelung des Magens mit *Heliobacter pylori* (HP) assoziiert, einem harnstoffabbauenden Bakterium, das auch als Campylobacter pylori bekannt ist. Der allgemeine Nachweis harnstoffabbauender Mikroorganismen, wie z.B. HP, wurde von Stuart et al. (*J. Bacteriol.* 1945, *49*, 437) und Christensen (*J. Bacteriol.* 1946, *52*, 461) beschrieben. Hinsichtlich der oben genannten gastrointestinalen Erkrankungen gilt HP mittlerweile als etablierte Ursache der chronischen Typ-B-Gastritis (Rauws et al., *Gastroenterology* 1988, *94*, 33). Im Falle der Ulkuskrankheit stellt HP einen Risikofaktor dar, der zum Duodenalulkusrezidiv prädisponiert (Coghlan et al., *Lancet II,* 1987, 1109). In den vergangenen Jahren wurde daher nach immer zuverlässigeren und vor allem praktikablen Nachweismethoden für HP gesucht. Hierbei hat die Analyse von Biopsieproben mit Ureasetests gegenüber der sehr sensitiven und spezifischen Kulturtechnik aus Gründen der leichten Handhabbarkeit und des geringen Aufwandes zunehmend an Bedeutung gewonnen.

So wurde z.B. von Christensen (*J. Bacteriol.* 1946, *52*, 461) ein Nährboden zur Differenzierung harnstoffabbauender Mikroorganismen beschrieben, der auf dem Abbau von Harnstoff durch Urease enthaltende Mikroorganismen beruht. Der Abbau von Harnstoff zu Kohlendioxid und Ammoniak führt zu einer alkalischen Reaktion des Mediums, die durch einen Farbumschlag des pH-Indikators Phenolrot von gelb nach rot angezeigt wird.

Ein modifizierter Schnelltest, der vornehmlich bei geringer *Heliobacter pylori* Besiedlung der Probe diagnostische Vorteile bietet, wurde von Malfertheiner beschrieben *(Acta Therapeutica,* 1988, *14*, 205).

Nach Börsch et al. (*Immun. Infekt.* 1989, *17*, 83-90) besitzt die kombinierte Untersuchung einer Antrum- und einer Korpusbiopsie gegenüber Einzelbiopsien generell eine überlegene diagnostische Sensitivität und Spezifität zum Nachweis der HP-Besiedelung des Magens und ist mit den Ergebnissen sensitiver Kulturtechniken durchaus vergleichbar. Die Kombination zweier antraler Ureasetests ist der kombinierten Untersuchung von Biopsien aus Antrum und Korpus deutlich unterlegen. Nach Börsch et al. stellen kombinierte Ureasetests einer Antrum/Korpus-Biopsie ein rationelles Diagnoseverfahren im Hinblick auf das Verhältnis von Aufwand und Ergebnis dar. Insbesondere gilt der Ureasetest als Methode der Wahl, wenn sensitive Kulturtechniken, die in der Durchführung im allgemeinen aufwendiger sind, nicht zur Verfügung stehen.

In der EP-A-204 438 wird ein Verfahren zur Bestimmung von Urease im Magenmaterial beschrieben, bei dem die verwendete Zusammensetzung aus Harnstoff, einem Bakterizid, welches das Wachstum Urease produzierender Organismen stark hindert, und einem pH-Indikator (z.B. Phenolrot) in einem Nährmedium (im wesentlichen bakteriologischer Agar) besteht. Die in den Figuren 1 bis 3 der EP 204 438 dargestellte Vorrichtung besteht aus einer rechteckigen Platte, in der sich ein kleiner, mit einer flexiblen Abdeckung verschließbarer Napf zur Aufnahme des beschriebenen Agars befindet.

Die bislang für Ureasetests beschriebenen und üblicherweise verwendeten Vorrichtungen bestehen im wesentlichen aus einfachen, verschließbaren Behältern, die der Aufnahme von nur einer Korpus- oder Antrum-Biopsie dienen. Diese Behälter müssen entsprechend markiert oder umständlich etikettiert werden, um nicht verwechselt zu werden. Da die verwendeten Behälter, z.B. (Eppendorf-)Röhrchen, sehr klein sind, ist eine Beschriftung mit den wichtigsten Patientendaten, mit klinischer Dokumentation bzw. mit Testzeiten und -ergebnissen nicht möglich. Bei einer entsprechenden Etikettierung wird aus ökonomischen Gründen in der Regel auf eine sorgfältige Beschriftung mit den wichtigsten Patientendaten verzichtet. Im routinemäßigen Laborbetrieb könnte es daher vorkommen, daß entnommene Biopsien falsch zugeordnet oder Biopsien mehrerer Patienten verwechselt werden.

Vorrichtungen, die die oben genannten Nachteile nicht aufweisen und der gleichzeitigen Aufnahme von Antrum- und Korpus-Biopsien für den kombinierten Ureasetest einer Antrum/Korpus-Biopsie dienen, sind im Stand der Technik bislang nicht bekannt.

Es ist daher Aufgabe der Erfindung, eine Vorrichtung zur Verfügung zu stellen, die die Durchführung von Ureasetests kombinierter Antrum/Korpus-Biopsien zur Diagnose von Gastrointestinalerkrankungen gestattet und eine deutliche, für den Fachmann unverwechselbare Zuordnung der entnommenen Biopsien zu Antrum und Korpus ermöglicht. Insbesondere ist Aufgabe der Erfindung, dem untersuchenden Arzt eine einfache, schnelle und sichere Handhabbarkeit der Vorrichtung zu bieten und eine Verwechslung der Biopsien mehrerer Patienten zu vermeiden.

Erfindungsgemäß dient zur Lösung dieser Aufgaben eine Vorrichtung zur Durchführung von Ureasetests kombinierter Antrum/Korpus-Biopsien zur Diagnose von Gastrointestinalerkrankungen mit einer Trägerptatte, einem an ihr befestigten oder befestigbaren, ein Substrat für den Ureasetest enthaltenden Behälter und einem Feld zur Aufnahme von Patientendaten und klinischer Dokumentation, **gekennzeichnet** durch eine auf die Trägerplatte (1) aufgebrachte schematische Darstellung (2) des Magens, wenigstens je eine Öffnung (3) in der Trägerplatte (1) jeweils an den dem Korpus und Antrum entsprechenden Stellen der schematischen Darstellung (2), wobei jede dieser Öffnungen die Öffnung eines Substrat enthaltenden, mit der Trägerplatte dauerhaft verbundenen, mit Deckel versehenen Behälters ist oder zur Aufnahme und Fixierung eines das Substrat enthaltenden verschließbaren Probengefäßes dient, und eine Auswertungsskala (4) zur Beurteilung des Ureasetests.

In einer Ausführungsform der Erfindung dienen die genannten Öffnungen der Vorrichtung zur Aufnahme und Fixierung handelsüblicher, verschließbarer Probengefäße, welche das für den Ureasetest verwendete Medium enthalten. In dieser Ausführungsform wird die schematische Darstellung des Magens entweder auf die Trägerplatte aufgedruckt oder in diese eingraviert.

Besonders bevorzugt ist aber eine Vorrichtung, bei der die genannten Öffnungen Öffnungen von Behältern sind, welche mit der Trägerplatte dauerhaft verbunden sind, ein Substrat für den durchzuführenden Ureasetest enthalten und mit einem Deckel verschlossen sind. Als Verschlußvorrichtung kann ein an der Trägerplatte befestigter Deckel verwendet werden. Alternativ wird vorzugsweise ein Adhäsionsverschluß in Form einer transparenten Folie dienen. Wird eine Adhäsionsfolie verwendet, kann sie mit der schematischen Darstellung des Magens bedruckt sein. Auf diese Weise wird die schematische Darstellung nicht durch Drucken oder Gravieren sondern in Form der bedruckten Folie auf die Trägerplatte aufgebracht. Die Öffnungen in der Trägerplatte können entweder durch getrennte Folienstücke oder aber mittels eines einzigen Folienstücks verschlossen sein. Vorzugsweise wird nur eine Folie zum Verschließen der Öffnungen verwendet. In am meisten bevorzugter Weise wird die schematische Darstellung des Magens direkt auf die Trägerplatte aufgedruckt oder in die Trägerplatte eingraviert, und die Öffnungen werden mit einem Deckel oder einer transparenten, unbedruckten Folie verschlossen. Auf diese Weise wird auch bei vollständigem Entfernen des Adhäsionsverschlusses gewährleistet, daß Antrum-bzw. Korpus-Biopsien richtig zugeordnet werden.

In einer weiteren Ausführungsform enthält die Trägerplatte an den Korpus und Antrum entsprechenden Stellen mehr als nur je eine Öffnung, was den Vorteil besitzt, daß gleichzeitig mehrere Antrum- und Korpus-Biopsien dem kombinierten Ureasetest unterzogen werden können. Mit den zusätzlichen Vergleichsbiopsien, die sich damit auf ein und derselben Trägerplatte untersuchen lassen, kann das Risiko falsch positiver Ureasetests deutlich verringert werden.

Die erfindungsgemäße Vorrichtung läßt sich in Kombination mit üblicherweise verwendeten Ureasetests verwenden. Vorzugsweise wird ein gelartiges Substrat verwendet, das in 1000 g 0,1 g Hefeextrakt, 0,1 g Kaliumdihydrogenphosphat, 0,06 g Phenolrot, 14,4 g Agar-Agar, 0,1 g Dextrose, 19,2 g Harnstoff, 2,9 ml Vitaminlösung und 0,1 ml Spurenelementlösung enthält.

Die genannte Vitaminlösung wird durch Lösen von 0,198 g D(+)Biotin, 1,977 g Nicotinsäure, 0,988 g Vitamin B1, 0,988 g *p*-Aminobenzoesäure, 0,494 g D-Pantothensäure-Natriumsalz, 4,942 g Pyridoxamindihydrochlorid, 1,977 g Vitamin B12 in 988,435 g destilliertem Wasser und anschließende Sterilfiltration hergestellt, und die genannte Spurenelementlösung wird durch Lösen von 0,049 g Bariumchlorid · 2 H₂O, 0,198 neutralem Kaliumiodid, 0,198 Kaliumbromid, 0.099 g Borsäure, 0,049 g Zinnchlorid, 0,049 Lithiumchlorid, 0,198 g Zinkchlorid · 2 H₂O, 0,099 g Kupfer(II)sulfat · 5 H₂O, 0,198 g Kobaltchlorid · 6 H₂O, 0,99 g Mangan(II)chlorid · 4 H₂O, 7,919 g Titriplex® III · 2 H₂O, 0,01 g Eisen(II)sulfat · 7 H₂O, 0,099 Natriummolybdat · H₂O, in 989,844 g bidestilliertem Wasser und anschließende Sterilfiltration hergestellt. Vorzugsweise werden reine Substanzen bzw. Substanzen für die Analytik (p.a.) verwendet.

Die nicht harnstoffhaltige Basis zubereitung wird autoklaviert und bei einer Temperatur von unter 62°C mit den thermolabilen Bestandteilen zur abfüllfertigen Zubereitung gemischt. Der pH-Wert des Substrates ist auf 6,0 ± 0,1 eingestellt.

Mit der erfindungsgemäßen Vorrichtung steht ein einfach zu handhabendes Hilfsmittel zur Diagnosestellung von Gastrointestinalerkrankungen zur Verfügung, welches die Durchführung der sensitiven und spezifischen kombinierten Ureasetests von Antrum/Korpus-Biopsien gestattet. Die auf die Trägerplatte aufgebrachte schematische Darstellung des Magens mit Behältern in den Korpus und Antrum darstellenden Bereichen gewährleistet eine einfache Probenzuordnung, die das Risiko einer versehentlichen Verwechslung bei der Zuordnung der entnommenen Biopsien zu Korpus und Antrum in einfacher Weise reduziert. Die erfindungsgemäße Vorrichtung ist in allen Fällen leicht handhabbar, und durch die auf einem einzigen Träger neben den Biopsien sowohl des Antrums als auch des Korpus vorliegenden Patientendaten und/oder der klinischen Dokumentation (z.B. Testzeiten und -ergebnisse) werden versehentliche Verwechslungen oder Vertauschungen auf einfache Art und Weise vermieden. Die auf der Trägerplatte befindliche Auswertungsskala gestattet die schnelle und eindeute Diagnosestellung durch direkten Vergleich mit auftretenden Farbänderungen der nebeneinanderliegenden Nährmedien, die Antrum- und Korpus-Biopsien enthalten. Die Biopsien bleiben auf einer Trägerplatte vereint bzw. fixiert und können auch nach 24 h gemeinsam auf etwaige Farbveränderungen des Ureasetests hin verglichen werden.

Die Form der erfindungsgemäßen Vorrichtung gestattet ein vorteilhaftes Lagern der Proben.

In der Zeichnung zeigen
- Fig. 1: eine perspektivische Darstellung einer ersten Ausführungsform nach der Erfindung,
- Fig. 2a: einen senkrechten Schnitt durch eine weitere Ausführungsform nach der Erfindung beim Verschließen mit einer Adhäsionsfolie,
- Fig. 2b: einen senkrechten Schnitt durch die in Fig. 2a dargestellte Vorrichtung nach Aufbringung der Adhäsionsfolie,
- Fig. 3: einen senkrechten Schnitt durch noch eine weitere Ausführungsform nach der Erfindung,
- Fig. 4: einen senkrechten Schnitt durch eine andere Ausführungsform nach der Erfindung und
- Fig. 5: eine Draufsicht auf eine weitere Ausführungsform der Erfindung.

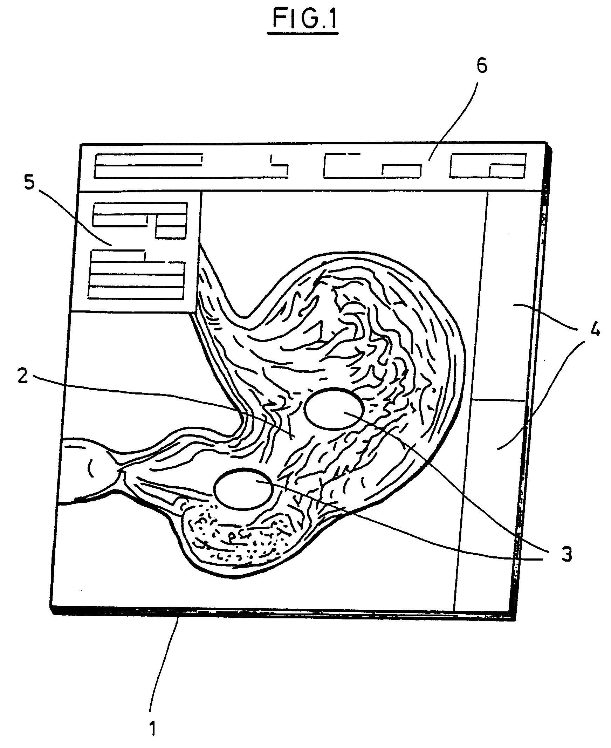

Fig. 1 zeigt eine Vorrichtung zur Durchführung kombinierter Ureasetests von Antrum/Korpus-Biopsien zur Diagnose von Gastrointestinalerkrankungen. Auf der Trägerplatte 1 befindet sich eine schematische Darstellung des Magens 2. An den dem Korpus und dem Antrum entsprechenden Stellen befindet sich jeweils eine Öffnung 3 zur Aufnahme eines im Substrat enthaltenen verschließbaren Probengefäßes. Zur Beurteilung des Testergebnisses befindet sich auf der Trägerplatte 1 eine Auswertungsskala 4, vorzugsweise mit farbigen Markierungen. Das auf der Trägerplatte befindliche Feld 5 dient zur Aufnahme von Patientendaten und zur klinischen Dokumentation, d. h. in diesem Feld kann die endgültige Beurteilung des kombinierten Ureasetests einer Antrum/Kopus-Biopsie eingetragen werden. Das Feld 6 enthält weitere Informationen zu diesem kombinierten Ureasetest, z. B. Hersteller oder allgemeine Erläuterungen.

Die in Fig. 1 dargestellte Vorrichtung dient zur Aufnahme von verschließbaren Behältern bzw. Röhrchen, die das zu verwendende Substrat für den Ureasetest enthalten.

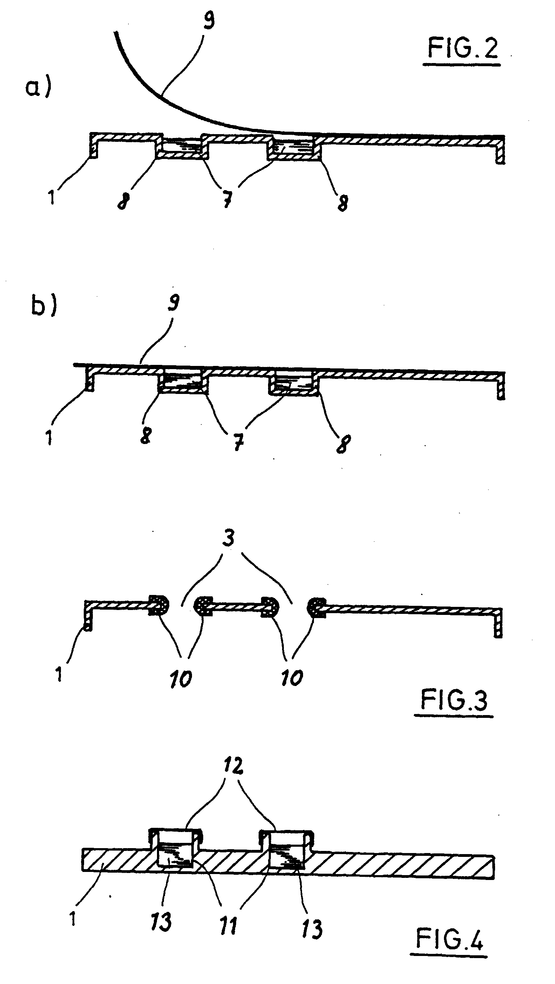

Fig. 2 zeigt Querschnitte einer Vorrichtung zur Durchführung kombinierter Ureasetests von Antrum/Korpus-Biopsien zur Diagnose von Gastrointestinalerkrankungen, bei der die Trägerplatte 1 der dargestellten Vorrichtung mit Substrat 7 gefüllte Behälter 8 besitzt, die sich mit Hilfe einer Folie 9 verschließen lassen. Die Lage der Behälter 8 in Fig. 2 ist mit derjenigen der Öffnungen 3 in Fig. 1 identisch.

Fig. 3 zeigt einen senkrechten Schnitt durch eine Vorrichtung zur Durchführung kombinierter Ureasetests von Antrum/Korpus-Biopsien zur Diagnose von Gastrointestinalerkrankungen, bei der in die Öffnungen 3 der Trägerplatte 1 die genannten Röhrchen eingesetzt werden, wobei die Röhrchen mit Hilfe in die Öffnungen eingesetzter Gummidichtungen bzw. -ringe 10 fixiert werden. Die Lager der Öffnungen 3 in Fig. 4 entspricht der Lage der Öffnungen 3 in Fig. 1 und gestattet so die eindeutige Zuordnung zu Korpus und Antrum.

Fig. 4 zeigt einen senkrechten Schnitt durch eine Vorrichtung, bei der die in die Trägerplatte 1 eingelassenen Behälter 11 mit einem über die Ebene der Trägerplatte 1 überstehenden Rand versehen sind. Der über die Ebene der Trägerplatte 1 überstehende Rand des Behälters 11 gestattet das Aufsetzen einer entsprechenden Verschlußvorrichtung, z. B. eines Deckels in Form eines Hütchens 12 oder besonders bevorzugt einer Schraubkappe mit zylindrischer Einlage, die einen innigen Kontakt der Biopsie mit dem zum Ureasetest verwendeten Substrat erzwingt, vorzugsweise die Biopsie in das Substrat hineindrückt. In diese Behälter 11 kann das zum Ureasetest verwendete Substrat 13 eingefüllt werden. Die Behälter 11 liegen an den in Fig. 1 dargestellten Orten der Öffnungen 3 und erlauben so die Zuordnung von Korpus und Antrum.

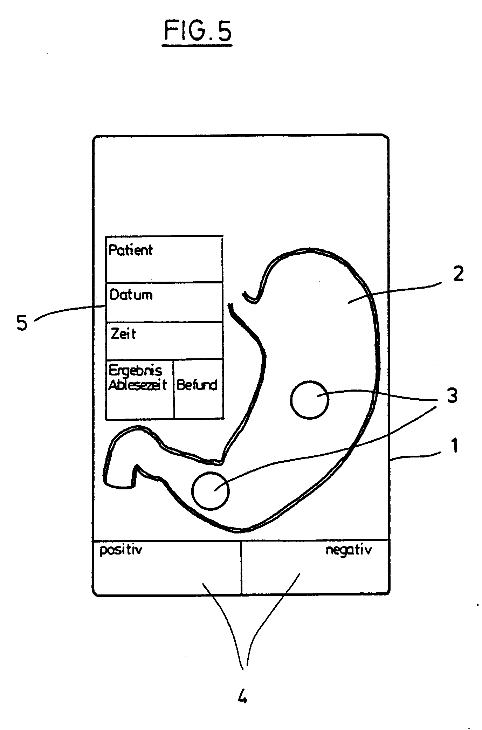

Fig. 5 zeigt eine Draufsicht auf eine besonders bevorzugte Ausführungsform der Erfindung. Die Numerierung entspricht Fig. 1.

## Patentansprüche

1. Vorrichtung zur Durchführung von Ureasetests kombinierter Antrum/Korpus-Biopsien zur Diagnose von Gastrointestinalerkrankungen mit einer Trägerplatte, einem an ihr befestigten oder befestigbaren, ein Substrat für den Ureasetest enthaltenden Behälter und einem Feld zur Aufnahme von Patientendaten und klinischer Dokumentation, **gekennzeichnet** durch eine auf die Trägerplatte (1) aufgebrachte schematische Darstellung (2) des Magens, wenigstens je eine Öffnung (3) in der Trägerplatte (1) jeweils an den dem Korpus und Antrum entsprechenden Stellen der schematischen Darstellung (2), wobei jede dieser Öffnungen die Öffnung eines Substrat enthaltenden, mit der Trägerplatte dauerhaft verbundenen, mit Deckel versehenen Behälters ist oder zur Aufnahme und Fixierung eines das Substrat enthaltenden verschließbaren Probengefäßes dient, und eine Auswertungsskala (4) zur Beurteilung des Ureasetests.

2. Vorrichtung nach Anspruch 1, **dadurch gekennzeichnet,** daß als Deckel ein Adhäsionsverschluß vorgesehen ist.

3. Vorrichtung nach den Ansprüchen 1 oder 2, **dadurch gekennzeichnet,** daß der Adhäsionsverschluß die schematische Darstellung des Magens trägt.

4. Vorrichtung nach Anspruch 1, **dadurch gekennzeichnet,** daß die Behälter einen über die Ebene der Trägerplatte überstehenden Rand besitzen.

5. Vorrichtung nach Anspruch 1 oder 4, **dadurch gekennzeichnet,** daß als Deckel ein Schraubverschluß vorgesehen ist, der eine zylindrische Einlage besitzt, die die genannten Biopsien in das Substrat drückt.

6. Vorrichtung nach Anspruch 1 oder 4, **dadurch gekennzeichnet,** daß die genannte schematische Darstellung des Magens auf die Trägerplatte aufgedruckt oder in die Trägerplatte eingraviert ist.

7. Vorrichtung nach einem der vorhergehenden Ansprüche , **dadurch gekennzeichnet,** daß für den Ureasetest ein gelartiges Substrat mit einem pH-Wert im Bereich zwischen 5,9 und 6,1 verwendet wird, das in 1000 g 0,1 g Hefeextrakt, 0,1 g Kaliumdihydrogenphosphat, 0,06 Phenolrot, 14,4 g Agar-Agar, 0,1 g Dextrose, 19,2 g Harnstoff, 2,9 ml Vitaminlösung und 0,1 ml Spurenelementlösung enthält.

8. Vorrichtung nach Anspruch 7, **dadurch gekennzeichnet,** daß die genannte Vitaminlösung durch Lösen von 0,198 g D (+) Biotin, 1,977 g Nicotinsäure, 0,988 g Vitmain B₁, 0,988 g p-Aminobenzoesäure, 0,494 g D-Pantothensäure-Natriumsalz, 4,942 g Pyridoxamindihydrochlorid, 1,977 g Vitamin B₁₂ in 988,435 g destilliertem Wasser und anschließende Sterilfiltration hergestellt wird und die genannte Spurenelementlösung durch Lösen von 0,049 g Bariumchlorid · 2 H₂O, 0,198 g neutralem Kaliumjodid, 0,198 g Kaliumbromid, 0,099 g Borsäure, 0,049 g Zinnchlorid, 0,049 g Lithiumchlorid, 0,198 g Zinkchlorid · 2 H₂O, 0,099 g Kupfer(II)sulfat · 5 H₂O, 0,198 g Kobaltchlorid · 6 H₂O, 0,99 g Mangan(II)chlorid · 4 H₂O, 7,919 g Tritriplex® III · 2 H₂O, 0,01 g Eisen(II)sulfat · 7 H₂O, 0,099 Natriummolybdat · H₂O, in 989,844 g bidestilliertem Wasser hergestellt wird.

## Claims

1. Apparatus for effecting urease tests of combined antrum/corpus biopsies for the diagnosis of gastro-intestinal illnesses, having a carrier board, a container which is fixed or can be fixed thereto and which contains a substrate for the urease test, and a panel for receiving patient data and clinical documentation, characterised by a diagrammatic representation (2) of the stomach which is applied to the carrier board (1), at least one respective opening (3) in the carrier board (1) at the respective locations in the diagrammatic representation (2), which correspond to the corpus and the antrum, wherein each of said openings is the opening of a substrate-containing container which is permanently connected to the carrier board and which is provided with a cover, or serves to receive and fix a closable specimen vessel containing the substrate, and an evaluation scale (4) for assessment of the urease test.

2. Apparatus according to claim 1 characterised in that an adhesion closure is provided as the cover.

3. Apparatus according to claim 1 or claim 2 characterised in that the adhesion closure carries the diagrammatic representation of the stomach.

4. Apparatus according to claim 1 characterised in that the containers have a rim which projects above the plane of the carrier board.

5. Apparatus according to claim 1 or claim 4 characterised in that the cover is a screw closure having a cylindrical insert which presses said biopsies into the substrate.

6. Apparatus according to claim 1 or claim 4 characterised in that said diagrammatic representation of the stomach is printed on to the carrier board or is engraved in the carrier board.

7. Apparatus according to one of the preceding claims characterised in that the urease test is implemented using a gel-type substrate with a pH-value in the range of between 5.9 and 6.1, which in 1000 g contains 0.1 g yeast extract, 0.1 g potassium dihydrogen phosphate, 0.06 g phenol red, 14.4 g agar-agar, 0.1 g dextrose, 19.2 g urea, 2.9 ml vitamin solution and 0.1 ml trace element solution.

8. Apparatus according to claim 7 characterised in that said vitamin solution is produced by dissolving 0.198 g D(+) biotin, 1.977 g nicotinic acid, 0.988 g vitamin B₁, 0.988 g p-aminobenzoic acid, 0.494 g D-pantothenic acid sodium salt, 4.942 g pyridoxamine dihydrochloride and 1.977 g vitamin B₁₂ in 988.435 g distilled water and then sterile filtration and said trace element solution is produced by dissolving 0.049 g barium chloride · 2H₂O, 0.198 g neutral potassium iodide, 0.198 g potassium bromide, 0.099 g boric acid, 0.049 g tin chloride, 0.049 g lithium chloride, 0.198 g zinc chloride · 2H₂O, 0.099 g copper (II) sulphate · 5H₂O, 0.198 g cobalt chloride · 6H₂O, 0.99 g manganese (II) chloride · 4H₂O, 7.919 g Tritriplex^{R}III·2H₂O, 0.01 g iron (II) sulphate ·7H₂O and 0.099 sodium molybdate·H₂O in 989.844 9 bidistilled water.

## Revendications

1. Procédé pour réaliser des tests d'uréase sur des biopsies combinées de l'antre et du corps pour le diagnostic de maladies gastro-intestinales avec une plaque support, un récipient qui y est ou qui peut y être fixé, contenant un substrat pour le test de l'uréase et un champ pour prendre les données des malades et la documentation clinique, caractérisé par une représentation schématique (2) de l'estomac déposée sur la plaque support (1), au moins une ouverture (3) dans la plaque support (1) toujours aux endroits de la représentation schématique (2) correspondant au corps et à l'antre, chacune de ces ouvertures étant l'ouverture d'un récipient contenant un substrat, fixé de façon durable à la plaque support et muni d'un couvercle, ou servant à accueillir et fixer un récipient d'échantillons scellable contenant le substrat, et par une échelle d'évaluation (4) pour juger du résultat du test de l'uréase.

2. Dispositif selon la revendication 1, caractérisé en ce qu'une fermeture adhérente sert de couvercle.

3. Dispositif selon la revendication 1 ou 2, caractérisé en ce que la fermeture adhérente porte la représentation schématique de l'estomac.

4. Dispositif selon la revendication 1, caractérisé en ce que les récipients possèdent un rebord faisant saillie au-dessus du plan de la plaque support.

5. Dispositif selon la revendication 1 ou 4, caractérisé en ce qu'il est fourni comme couvercle une fermeture à vis qui possède une pièce intercalaire cylindrique pressant les biopsies mentionnées dans le substrat.

6. Dispositif selon la revendication 1 ou 4, caractérisé en ce que la représentation schématique de l'estomac mentionnée est imprimée sur la plaque support ou gravée dans la plaque support.

7. Dispositif selon l'une des revendications précédentes, caractérisé en ce qu'on utilise pour le test de l'uréase un substrat de type gel avec un pH compris entre 5,9 et 6,1, qui dans 1000 g contient 0,1 g d'extrait de levure, 0,1 g de phosphate diacide de potassium, 0,06 g de rouge de phénol, 14,4 g d'agar-agar, 0,1 g de dextrose, 19,2 g d'urée, 2,9 ml de solution de vitamine et 0,1 ml de solution d'oligo-éléments.

8. Dispositif selon la revendication 7, caractérisé en ce qu'on prépare la solution de vitamine mentionnée par dissolution de 0,198 g de D(+)-biotine, 1,977 g d'acide nicotinique, 0,988 g de vitamine B₁, 0,988 g d'acide p-aminobenzoïque, 0,494 g de sel de sodium de l'acide D-panthothénique, 4,942 g de dichlorhydrate de pyridoxamine, 1,977 g de vitamine B₁₂ dans 988,435 g d'eau distillée, puis par filtration stérile, et en ce qu'on prépare la solution d'oligo-éléments mentionnée par dissolution de 0,049 g de chlorure de baryum. 2 H₂O, 0,198 g d'iodure de potassium neutre, 0,198 g de bromure de potassium, 0,099 g d'acide borique, 0,049 g de chlorure d'étain, 0,049 g de chlorure de lithium, 0,198 g de chlorure de zinc. 2 H₂O, 0,099 g de sulfate de cuivre (II). 5 H₂O, 0,198 g de chlorure de cobalt. 6 H₂O, 0,99 g de chlorure de manganèse (II). 4 H₂O, 7,919 g de Tritriplex® III. 2 H₂O, 0,01 g de sulfate de fer (II). 7 H₂O, 0,099 g de molybdate de sodium. H₂O, dans 989,844 g d'eau bidistillée.
